# EUROPÄISCHE PATENTSCHRIFT

(11) **EP 0 810 883 B1**
(45) Veröffentlichungstag und Bekanntmachung des Hinweises auf die Patenterteilung: **31.07.2002**
(21) Anmeldenummer: 96903881.9
(22) Anmeldetag: 21.02.1996
(51) Int. Cl.: A61K 51/08, A61P 35/00

(54) **KONJUGAT ENTHALTEND DTPA UND ALBUMIN ZUR INDIVIDUELLEN DOSIERUNG VON ARZNEIMITTELN**
CONJUGATE COMPRISING DTPA AND ALBUMIN FOR INDIVIDUAL MEDICAMENT DOSING
CONJUGUE CONTENANT DTPA ET ALBUMINE POUR LE DOSAGE INDIVIDUEL DE MEDICAMENTS

(30) Priorität: 21.02.1995 DE 19505960
(43) Veröffentlichungstag der Anmeldung: 10.12.1997
(73) Patentinhaber: Deutsches Krebsforschungszentrum Stiftung des öffentlichen Rechts, 69120 Heidelberg (DE)
(72) Erfinder: SINN, Hannsjörg, D-69118 Wiesloch (DE); MAIER-BORST, Wolfgang, D-69221 Dossenheim (DE); SCHRENK, Hans-Hermann, D-67278 Zeiskam (DE); STEHLE, Gerd, D-69123 Heidelberg (DE)
(74) Vertreter: Schüssler, Andrea, Dr.
(86) Internationale Anmeldenummer: DE9600267
(87) Internationale Veröffentlichungsnummer: WO9625956

(56) Entgegenhaltungen:
- EP-A- 0 217 577
- EP-A- 0 243 929
- WO-A-89/00062
- WO-A-89/05853
- WO-A-91/01144
- WO-A-91/14459
- WO-A-93/02105
- WO-A-93/02192
- WO-A-94/08624
- WO-A-95/19791
- WO-A-95/29707
- BE-A- 848 379
- DE-A- 4 435 087
- GB-A- 2 268 494
- US-A- 4 339 426
- NUCLEAR MEDICINE AND BIOLOGY, Bd. 20, Nr. 2, 1.Februar 1993, Seiten 231-237, XP000336605 SUSUMU NAKAJIMA ET AL: "111IN-LABELED MN-METALLOPORPHYRIN FOR TUMOR IMAGING"
- INTERNATIONAL JOURNAL OF RADIATION APPLICATIONS AND INSTRUMENTATION PART B: NUCLEAR MEDICINE AND BIOLOGY, Bd. 17, Nr. 8, 1.Januar 1990, Seiten 819-827, XP000166070 SINN H ET AL: "DESIGN OF COMPOUNDS HAVING AN ENHANCED TUMOUR UPTAKE, USING SERUM ALBUMIN AS A CARRIER. PART I"
- J. LABELLED COMPD. RADIOPHARM., Bd. 32, Januar 1993, XP002008742 H. SINN ET AL.: "RADIOACTIVE LABELLED PHOTOSENSITIZERS FOR TUMOR DIAGNOSTIC AND PHOTODYNAMIC THERAPY (PDT)"
- CHEMICAL ABSTRACTS, vol. 121, no. 4, 25.Juli 1994 Columbus, Ohio, US; abstract no. 42716, KATO, AKIHISA ET AL: "Anti-retroviral formulations containing dyes" XP002016958 & JP,A,06 016 561 (TSUMURA & CO, JAPAN)

## Beschreibung

Die Erfindung betrifft ein Konjugat zur individuellen Dosierung von Arzneimitteln, Verfahren zur Herstellung eines solchen Konjugats sowie dessen Verwendung.

Seit langem ist es ein großes Bedürfnis, Arzneimittel individuell zu dosieren, d.h. ihre Dosis genau auf den Therapieverlauf bei einem Patienten abzustellen. Dieses Bedürfnis liegt insbesondere bei Verwendung von Chemotherapeutika vor. Viele Versuche wurden unternommen, vorstehendes zu erreichen. Bisher waren allerdings die Versuche nur wenig erfolgreich.

Nakajima et al. (Nuclear Medicine and Biology, Bd. 20, Nr. 2, 1993, S. 231-237) beschreiben ein Konjugat umfassend ein Mn-enthaltendes Porphyrin und DTPA, welches an ¹¹¹In gebunden ist. Dieses Konjugat wird für die Tumordarstellung verwendet. Allerdings besitzt es keine Photosensitivität. Weiterhin beschreibt D1 ein ¹¹¹In-markiertes Ga-Metallporphyrin-Konjugat, das phototoxisch ist.

WO-A-94/08624 beschreibt ein Polymer, das Poly(alkylenoxid) enthält, welches an einen Chelator, wie EDTA oder DTPA, gebunden ist, wobei das Polymer weiter ein zytotoxisches Mittel, wie Antibiotika, alkylierende Mittel oder Antimetaboliten, aufweist. Die Polymere werden in der Diagnose und Therapie von Erkrankungen eingesetzt.

WO-A-91/01144 beschreibt ein Konjugat aus einem biologisch aktiven Peptid und einer Chelatgruppe, z.B. EDTA, DTPA, TTHA, die eine nachweisbare Verbindung, z.B. Gd³⁺, aufweist.

EP-A-0 217 577 beschreibt Hapten-modifizierte Mittel, die Metallkomplexe von Benzyl-EDTA umfassen, z.B. Mitomycin C-aminobenzyl-EDTA-thioharnstoff. Diese werden zur Diagnose und Therapie von Tumoren und Infektionskrankheiten verwendet.

EP-A-0 243 929 beschreibt Protein- und Polypeptid-Derivate, die mit einer Reportergruppe oder einem zytotoxischen Mittel konjugiert sind. Die Reportergruppe kann ein Metallderivat von DTPA sein. Auch diese Verbindungen werden zur Diagnose eingesetzt.

Sinn et al. (Nuclear Medicine and Biology, 1990, S. 819-827) beschreiben Verbindungen, die mit radioaktivem Iod markiert sind und an Albumin gebunden sind. Diese Verbindungen weisen eine erhöhte Aufnahme in Tumoren auf.

Sinn et al. (Labelled Comp. Radiopharm. 1993, S. 398-399) beschreiben, daß Albumin und das synthetische Polymer PEOM als Trägersystem für Photosensitizer für die Tumordiagnose und photodynamische Therapie verwendet werden.

Der vorliegenden Erfindung liegt somit die Aufgabe zugrunde, ein Mittel bereitzustellen, mit dem Arzneimittel individuell dosiert werden können.

Erfindungsgemäß wird dies durch ein Konjugat erreicht, das einen Wirkstoff, eine eine Bindungsstelle für Metallverbindungen aufweisende Verbindung und einen Träger umfaßt.

Der Ausdruck "Wirkstoff" umfaßt Substanzen jeglicher Art, die zur Therapie und/oder Diagnose einer Erkrankung, insbesondere einer Tumorerkrankung, Infektionserkrankung, Hauterkrankung und/oder Erkrankung des Immunsystems, verwendet werden können.

Beispiele solcher Substanzen sind Chemotherapeutika, z.B. Antibiotika, Virostatika, Antiprotozoenmittel und Zytostatika. Beispiele der Antibiotika sind Sulfonamide, Tetracycline, z.B. 7-Chlortetracyclin, Fusidinsäure, Gyrasehemmstoffe, z.B. Chinolone, Amphotericin, Isoniazid, Pyrazin 2-carbonsäure und Pyrazinamid. Beispiele der Virustatika sind Amantadin und Rimantadin. Beispiele von Antiprotozoenmittel sind Mefloquin und Primaquin. Beispiele für Zytostatika sind Anthrazykline, z.B. Doxorubicin, Topoisomerase-Hemmstoffe, Mitomycin A und C, Bleomycinsäure, Chlorambucil, Melphalan und Folsäureantagonisten, z.B. Methotrexat.

Weiterhin kann eine solche Substanz ein Aminoanthrachinon, wie Cellitonblau und Säureschwarz, sein.

Ferner kann eine solche Substanz eine photoaktive Substanz sein, z.B. ein Porphyrin, wie o-, m-, und/oder p-Tetrahydroxyphenylporphin, o-, m- und/oder p-Tetracarboxyphenylporphin und o-, m- und/oder p-Tetrasulfophenylporphin, ein Chlorin, wie o-, m- und/oder p-Tetrahydroxyphenylchlorin, o-, m- und/oder p-Tetracarboxyphenylchlorin und o-, m-, und/oder p-Tetrasulfophenylchlorin, oder ein Bakteriochlorin, o-, m- und/oder p-Tetrahydroxyphenylbakteriochlorin wie o-, m- und/oder p-Tetracarboxyphenylbakteriochlorin und o-, m-, und/oder p-Tetrasulfophenylbakteriochlorin.

Desweiteren kann eine solche Substanz ein Kontrastmittel für die Fluorspektroskopie, z.B. Trifluoressigsäure, Kernspintomographie oder Szintigraphie sein.

Von vorstehenden Substanzen bzw. Analoga oder Derivaten davon liegen ein oder mehrere in einem erfindungsgemäßen Konjugat vor. Bei mehreren, können diese gleich oder verschieden voneinander sein.

Der Ausdruck "Bindungsstelle für Metallverbindungen aufweisende Verbindung" bedeutet eine Verbindung, die Bindungsstellen für Metallverbindungen aufweist. Beispiele solcher Bindungsstellen sind Hydroxylgruppen, insbesondere an C-Atome gebundene Hydroxylgruppen, Carbonyl- und Carboxylgruppen. Die Verbindung kann eine oder mehrere Bindungsstellen aufweisen. Vorzugsweise hat die Verbindung mindestens 2, besonders bevorzugt 3 bis 6 Bindungsstellen. Liegen mehrere Bindungsstellen vor, so können diese gleich oder verschieden voneinander sein. Eine vorstehende Verbindung ist Diethylentriaminpentaacetat (DTPA) und Derivate davon.

Von vorstehenden Verbindungen liegen eine oder mehrere in einem erfindungsgemäßen Konjugat vor. Bei mehreren, können diese gleich oder verschieden voneinander sein. Das Vorliegen mehrerer der vorstehenden Verbindungen begünstigt die Wasserlöslichkeit des Konjugats und seine Fähigkeit, Metallverbindungen zu binden.

In einer bevorzugten Ausführungsform weist ein erfindungsgemäßes Konjugat eine nachweisbare Metallverbindung auf. Eine solche kann ein oder mehrere nachweisbare Metalle und/oder Metallionen enthalten oder aus solchen bestehen. Beispiele solcher Metalle sind Zn, Cu, Co, Fe, Ni, Pt, Gd und In, die vorzugsweise 2- oder 3-wertig sind, wobei Gd³⁺ besonders bevorzugt ist. Die Metalle und/oder Metallionen können radioaktiv sein, wie ¹¹¹In.

Erfindungsgemäß weist ein erfindungsgemäßes Konjugat einen Träger auf. Ein solcher Träger ist das Protein Albumin, insbesondere humanes Serumalbumin (HSA).

Ein erfindungsgemäßes Konjugat kann einen oder mehrere vorstehender Träger aufweisen. Liegen mehrere Träger vor, können diese gleich oder verschieden voneinander sein.

In einem erfindungsgemäßen Konjugat können ein oder mehrere Wirkstoffe mit einer oder mehreren Verbindungen verbunden sein, die Bindungsstellen für Metallverbindungen aufweisen. Ferner können die Wirkstoffe und/oder die Verbindungen untereinander verbunden sein.

Ein solches Konjugat kann auch eine oder mehrere nachweisbare Metallverbindungen enthalten. Diese können von der Bindung zwischen dem oder den Wirkstoffen und der oder den Verbindungen ausgenommen sein oder diese darstellen.

Ein vorstehendes Konjugat enthält ferner einen oder mehrere Träger, der mit der oder den Verbindungen und/oder der oder den Metallverbindungen verbunden ist.

Vorstehende Komponenten des erfindungsgemäßen Konjugats sind als Edukte angegeben. Im Konjugat liegen Sie derivatisiert vor.

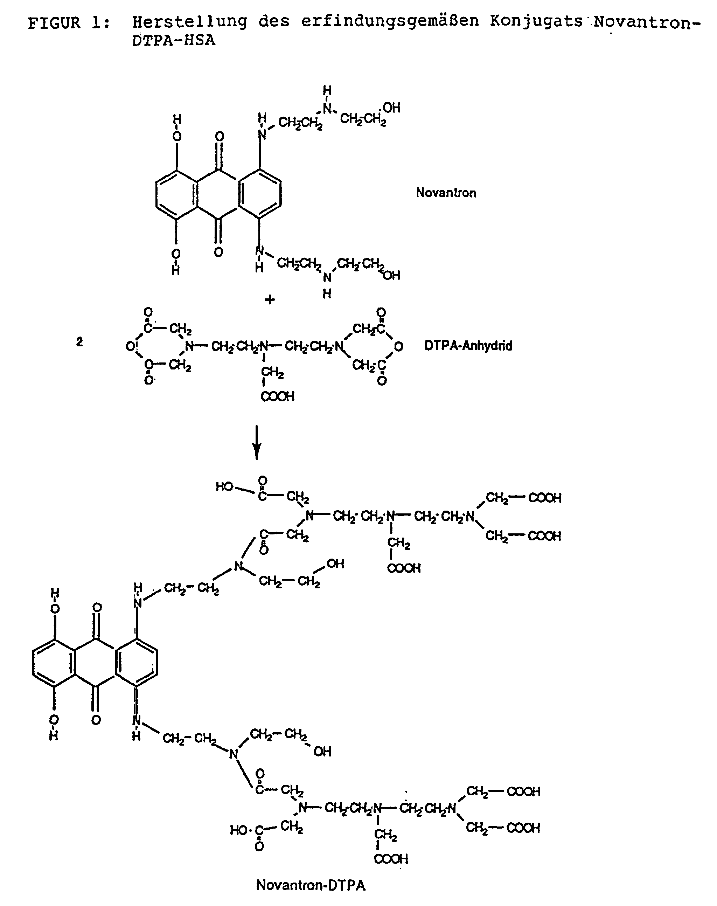

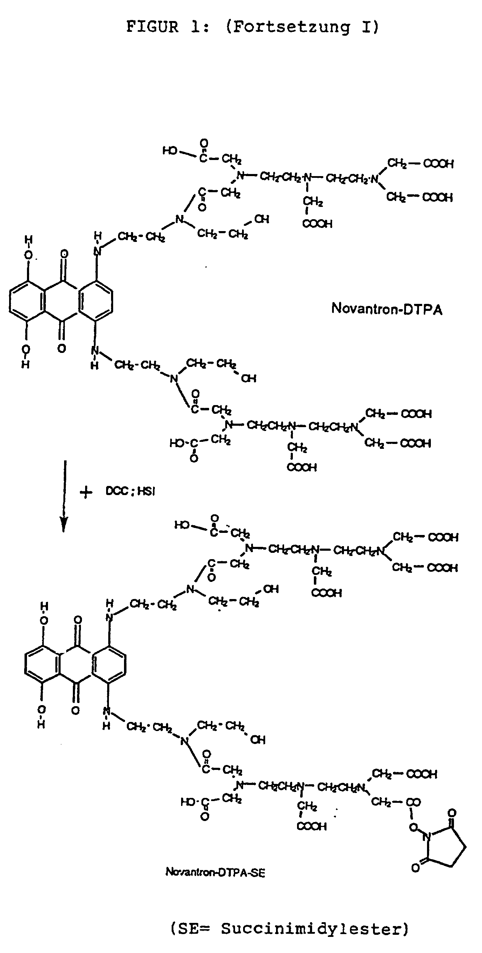

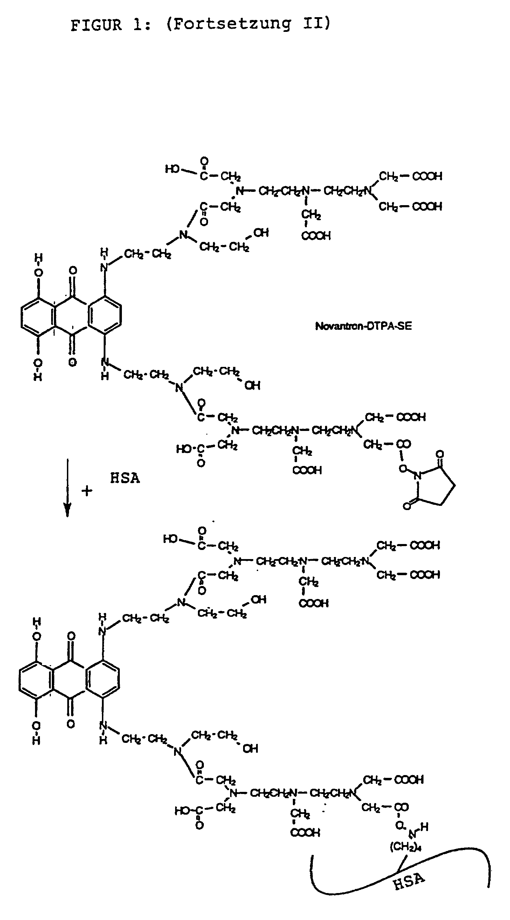

Bevorzugte erfindungsgemäße Konjugate sind in den Figuren 1 - 6 dargestellt.

Erfindungsgemäß wird auch ein Verfahren zur Herstellung eines vorstehenden Konjugats bereitgestellt. In einem solchen Verfahren werden übliche Umsetzungen der Chemie, wie Aktivierung einer Säuregruppe und Verknüpfung der aktivierten Säuregruppe mit einer Aminogruppe, durchgeführt. Hierzu wird auf die Herstellung der Konjugate in den Beispielen 1 und 2 sowie in den Figuren 1 -6 verwiesen.

Erfindungsgemäße Konjugate werden in markierter und unmarkierter Form bereitgestellt. Beide Formen eignen sich gut für therapeutische Zwecke. Günstig ist es, die markierte Form zu verschiedenen Zeiten der Therapie zu verabreichen, wodurch die Aufnahme und Verteilung des erfindungsgemäßen Konjugats im Körper und seine Wirkung bestimmt werden kann. Damit ist es möglich, das Konjugat individuell zu dosieren. Dies stellt einen großen Vorteil dar, der sich insbesondere in der Chemotherapie auswirkt. Durch die individuelle Dosierung des Konjugats können die Nebenwirkungen auf gesundes Gewebe minimiert werden. Dies wird insbesondere erreicht, wenn das erfindungsgemäße Konjugat einen Träger enthält, der eine Anreicherung des Konjugats in bestimmten Geweben, z.B. Tumoren oder Entzündungsherden, bewirkt. Somit ermöglicht die vorliegende Erfindung eine ziel- und dosisgenaue Verabreichung von Arzneimitteln.

### Kurze Beschreibung der Zeichnungen

- Fig. 1: zeigt die Herstellung des erfindungsgemäßen Konjugats Novantron-DTPA-HSA,
- Fig. 2: zeigt die Herstellung des erfindungsgemäßen Konjugats Cellitonblau-DTPA-HSA,
- Fig. 3: zeigt die Wachstumsinhibition von Tumorzellen durch Verabreichung von Cellitonblau-DTPA-HSA bzw. Novantron-DTPA-HSA, und
- Fig. 4: zeigt die Verteilung von ¹¹¹In- Novantron-DTPA-HSA über der Tumor-, Herz- und Leberregion.

Die folgenden Beispiele erläutern die Erfindung.

### Beispiel 1: Herstellung des erfindungsgemäßen Konjugats Novantron-DTPA-HSA

Die Herstellung des Konjugats und seine Struktur sind in Figur 1 gezeigt.

Novantron wurde in einer Konzentration von 10 mg/mℓ in 0,17 M Bic gelöst. Zu dieser Lösung wurde portionsweise DTPA-Anhydrid zugegeben, bis dünnschichtchromatographisch unter Standard-Bedingungen kein freies Novantron mehr nachweisbar war.

| DC: Standardbedingungen | | |
|---|---|---|
| Platten: | Kieselgel 60 (5x20 cm) ohne Fluoreszenzindikator | |
| Laufmittel: | Etac. 70; MeOH 30 (v/v) | |
| Rf. | Novantron | 0,58 - 0,62 |
| | Novantron-DTPA | 0,0 |

Anschließend wurde die Lösung mit 2 N HCℓ versetzt bis sich ein blauer Niederschlag bildete (etwa bei pH 2). Das so erhaltene Gemisch wurde nachfolgend am Rotationsverdampfer zur Trockne eingeengt.

Der nach dem Einengen am Rotationsverdampfer erhaltene Rückstand wurde in DMF gelöst und mit einem zweifachen molaren Überschuß an Dicyclohexylcarbodiimid (DCC) und einem zehnfachen Überschuß an N-Hydroxysuccinimid (HSI) versetzt. Nach etwa 12 Stunden erfolgte die Zugabe von HSA (10 mg/mℓ in 0,17 M NaHCO₃). Das Reaktionsgemisch wurde etwa 30 Minuten stehengelassen. Danach wurde in üblicher Weise DCC, DCHH, DMF und HSI abgetrennt. Die Reinheit wurde mittels HPLC und DC überprüft. Es wurde Novantron-DTPA-HSA erhalten.

### Beispiel 2: Herstellung des erfindungsgemäßen Konjugats Cellitonblau-DTPA-HSA

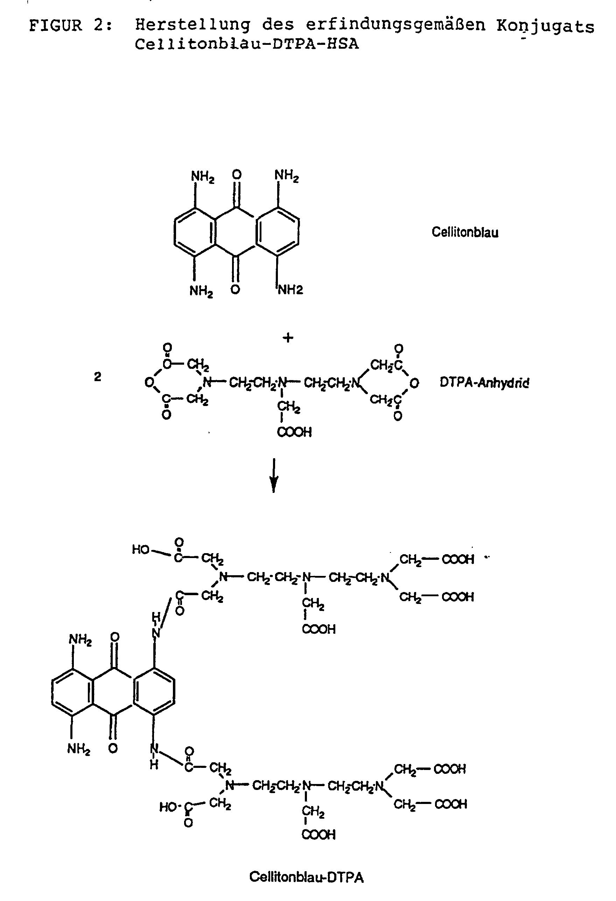

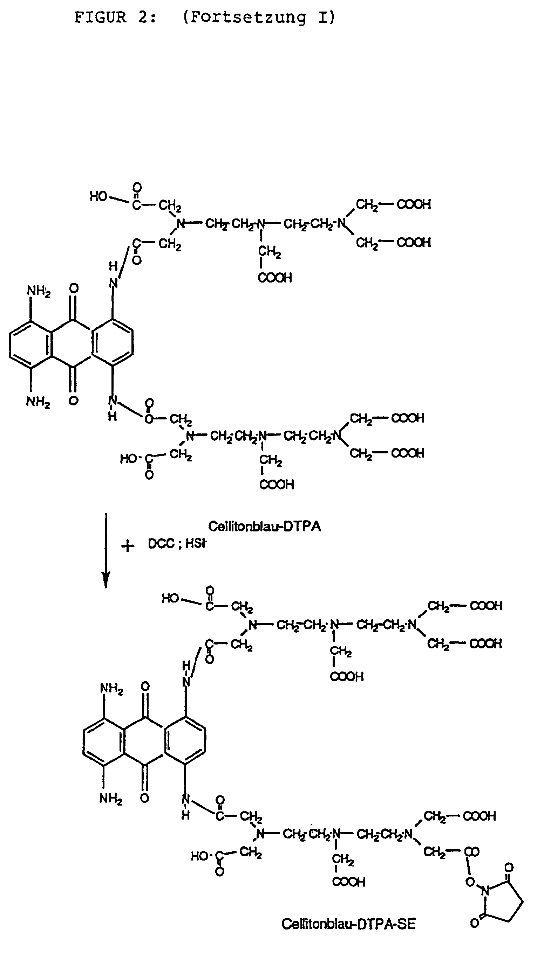

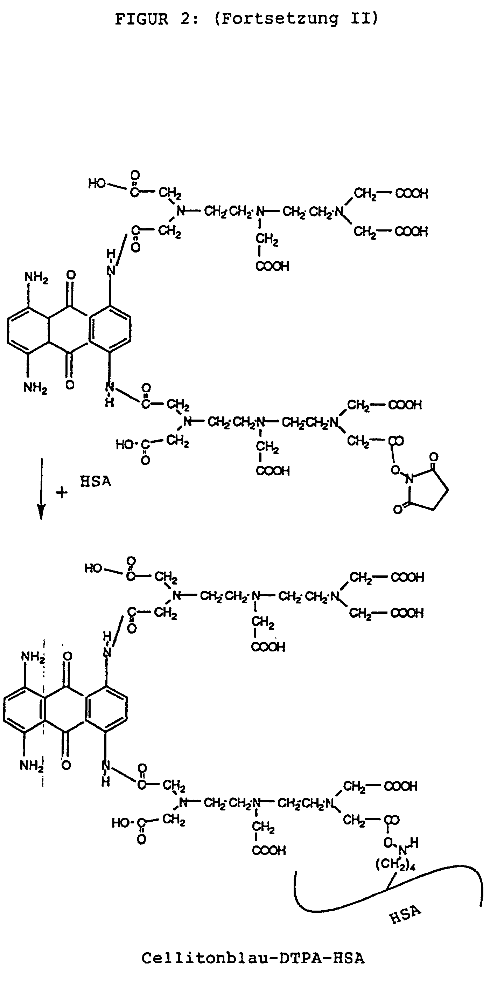

Die Herstellung des Konjugats und seine Struktur sind in Figur 2 gezeigt.

Cellitonblau (1,4,5,8-Tetraaminoanthrachinon, FG. 268,28) wurde in einer Konzentration von 20 mg/mℓ in DMF gelöst. Unter Rühren wurde solange DTPA-Anhydrid zugegeben, bis dünnschichtchromatographisch kein freies Cellitonblau mehr nachweisbar war.

| DC: Standardbedingungen: | | |
|---|---|---|
| Rf.-Werte | Cellitonblau | 0,62-0,66 |
| | Cellitonblau-DTPA | 0,0 |

Die Reinigung der blauviolett gefärbten Substanz erfolgte über Ultrafiltration (YC 05), wobei der pH der Lösung auf etwa 6,5 eingestellt wurde. Anschließend wurde die Lösung mit 2 N HCℓ angesäuert (pH 2,0-2,5) und unmittelbar darauf am Rotationsverdampfer zur Trockne eingeengt. Die Probe wurde anschließend in Methanol wieder gelöst und erneut am Rotationsverdampfer eingeengt, um Restmengen von Wasser zu entfernen. Dies wurde mindestens dreimal durchgeführt. Die Umsetzung des Cellitonblau-DTPA mit DCC und HSI erfolgte wie in Beispiel 1 beschrieben. Nach 12 bis 14 Stunden erfolgte die Umsetzung mit HSA. Nach einer Reaktionszeit von etwa 30 Minuten wurden Begleitstoffe, wie in Beispiel 1 beschrieben, abgetrennt. Die Reinheit wurde mittels DC und HPLC überprüft. Es wurde Cellitonblau-DTPA-HSA erhalten.

### Beispiel 3: Wachstumsinhibition von Tumorzellen durch Verabreichung von Cellitonblau-DTPA-HSA bzw. Novantron-DTPA-HSA

Die Konjugate Cellitonblau-DTPA-HSA und Novantron-DTPA-HSA sowie HSA als Kontrolle wurden jeweils mit Walker-256-Tumorzellen unter üblichen Bedingungen inkubiert. Nach 24 bzw. 48 Stunden wurde in üblicher Weise die Zellzahl pro mℓ bestimmt.

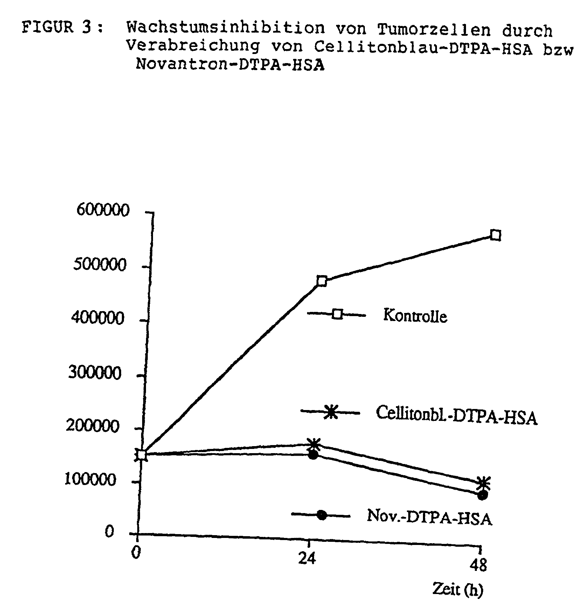

Aus Figur 3 geht hervor, daß jedes der erfindungsgemäßen Konjugate die Proliferation von Tumorzellen inhibiert.

### Beispiel 4: Verteilung von ¹¹¹In-Novantron-DTPA-HSA über der Tumor-, Herzund Leberregion

Das erfindungsgemäße Konjugat wurde durch eine intravenöse Injektion einer tumortragenden Ratte (Walker-256 Karzinosarkom) verabreicht. In üblicher Weise wurde szintigraphisch die prozentuale Aufnahme, bezogen auf die Gesamtmenge an verabreichtem Konjugat, gemessen.

Desweiteren wurde vorstehendes Konjugat narkotisierten Ratten verabreicht und in üblicher Weise szintigraphisch die Abnahme des erfindungsgemäßen Konjugats in der Herz- bzw. Leberregion gemessen.

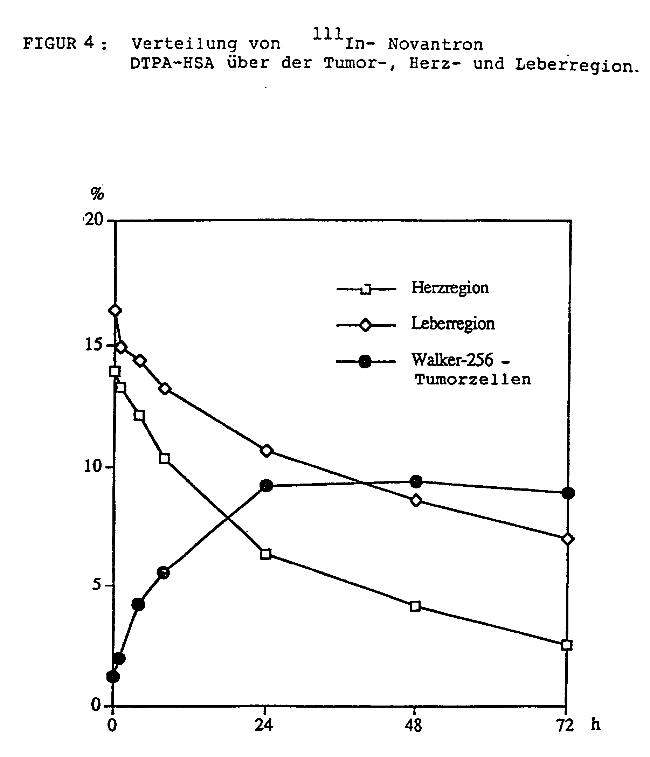

Aus Figur 4 geht hervor, daß sich das erfindungsgemäße Konjugat im Tumor anreichert, nicht jedoch im Bereich des Herzens oder der Leber.

## Patentansprüche

1. Konjugat umfassend einen Wirkstoff, eine eine Bindungsstelle für Metallverbindungen aufweisende Verbindung und einen Träger, wobei der Träger Albumin ist und die eine Bindungsstelle für Metallverbindungen aufweisende Verbindung DTPA oder ein Derivat davon ist, welche sich zwischen dem Wirkstoff und dem Albumin befindet.

2. Konjugat nach Anspruch 1, **dadurch gekennzeichnet, daß** mehrere Wirkstoffe vorliegen.

3. Konjugat nach Anspruch 1 oder 2, **dadurch gekennzeichnet, daß** mehrere Bindungsstellen vorliegen.

4. Konjugat nach Anspruch 3, **dadurch gekennzeichnet, daß** 2 bis 6 Bindungsstellen vorliegen.

5. Konjugat nach einem der Ansprüche 1 bis 4, **dadurch gekennzeichnet, daß** mehrere Verbindungen vorliegen, die eine Bindungsstelle für Metallverbindungen aufweisen.

6. Konjugat nach einem der Ansprüche 1 bis 5, **dadurch gekennzeichnet, daß** das Konjugat weiter eine nachweisbare Metallverbindung aufweist.

7. Konjugat nach Anspruch 6, **dadurch gekennzeichnet, daß** die Metallverbindung ein nachweisbares Metall und/oder Metallion enthält oder aus solchen besteht.

8. Konjugat nach Anspruch 7, **dadurch gekennzeichnet, daß** das Metall oder Metallion radioaktiv ist.

9. Konjugat nach einem der Ansprüche 1 bis 8, **dadurch gekennzeichnet, daß** mehrere Träger vorliegen.

10. Verfahren zur Herstellung eines Konjugats nach Ansprüchen 1 bis 9, **dadurch gekennzeichnet, daß** ein Wirkstoff mit einer eine Bindungsstelle für Metallverbindungen aufweisenden Verbindung und einem Träger sowie ggfs. mit einer nachweisbaren Metallverbindung in üblicher Weise verbunden wird, wobei die eine Bindungsstelle für Metallverbindungen aufweisende Verbindung DTPA oder ein Derivat davon ist und der Träger Albumin ist.

11. Verwendung eines Konjugats nach einem der Ansprüche 1 bis 9 zur Herstellung eines therapeutischen oder diagnostischen Mittels zur Therapie und/oder Diagnose einer Erkrankung.

12. Verwendung nach Anspruch 11, wobei die Erkrankung eine Tumorerkrankung, Infektionserkrankung, Hauterkrankung und/oder Erkrankung des Immunsystems ist.

## Claims

1. A conjugate comprising an active substance, a compound having a biding site for metal compounds and a carrier, wherein the carrier is albumin and the compound having a binding site for metal compounds is DTPA or a derivative thereof, which is located between the active substance and albumin.

2. The conjugate according to claim 1, **characterized in that** several active substances are present.

3. The conjugate according to claim 1 or 2, **characterized in that** several binding sites are present.

4. The conjugate according to claim 3, **characterized in that** 2 to 6 binding sites are present.

5. The conjugate according to any of claims 1 to 4, **characterized in that** several compounds are present which have a binding site for metal compounds.

6. The conjugate according to any of claims 1 to 5, **characterized in that** the conjugate also has a detectable metal compound.

7. The conjugate according to claim 6, **characterized in that** the metal compound contains, or consists of, a detectable metal and/or metal ion.

8. The conjugate according to claim 7, **characterized in that** the metal or metal ion is radioactive.

9. The conjugate according to any of claims 1 to 8, **characterized in that** several carriers are present.

10. A method of producing a conjugate according to claims 1 to 9, **characterized in that** an active substance is connected as usual with a compound having a binding site for metal compounds and a carrier as well as, where appropriate, with a detectable metal compound, the compound having a binding site for metal compounds being DTPA or a derivative thereof and the carrier being albumin.

11. Use of a conjugate according to any of claims 1 to 9 for the production of a therapeutic or diagnostic agent for treating and/or diagnosing a disease.

12. Use according to claim 11, wherein the disease is a tumoral disease, infectious disease, skin disease and/or a disease of the immune system.

## Revendications

1. Conjugué comprenant une substance active, un composé présentant un site de liaison pour des composés métalliques et un support, dans lequel le support est l'albumine et le composé présentant un site de liaison pour des composés métalliques est le DTPA ou un dérivé de DTPA, se trouvant entre la substance active et l'albumine.

2. Conjugué suivant la revendication 1, **caractérisé en ce que** plusieurs substances actives sont présentes.

3. Conjugué suivant la revendication 1 ou 2, **caractérisé en ce que** plusieurs sites de liaison sont présents.

4. Conjugué suivant la revendication 3, **caractérisé en ce que** 2 à 6 sites de liaison sont présents.

5. Conjugué suivant l'une des revendications 1 à 4, **caractérisé par** la présence de plusieurs composés qui présentent un site de liaison pour des composés métalliques.

6. Conjugué suivant l'une des revendications 1 à 5, **caractérisé en ce qu'**il présente en outre un composé métallique détectable.

7. Conjugué suivant la revendication 6, **caractérisé en ce que** le composé métallique contient un métal et/ou un ion métallique détectables ou en est constitué.

8. Conjugué suivant la revendication 7, **caractérisé en ce que** le métal ou l'ion métallique est radio-actif.

9. Conjugué suivant l'une des revendications 1 à 8, **caractérisé en ce que** plusieurs supports sont présents.

10. Procédé de production d'un conjugué suivant les revendications 1 à 9, **caractérisé en ce qu'**on lie de manière classique une substance active à un composé présentant un site de liaison pour des composés métalliques et à un support ainsi que, le cas échéant, à un composé métallique détectable, le composé présentant un site de liaison pour des composés métalliques étant le DTPA ou un dérivé de DTPA et le support étant l'albumine.

11. Utilisation d'un conjugué suivant l'une des revendications 1 à 9, pour la préparation d'un moyen thérapeutique ou diagnostique destiné au traitement et/ou au diagnostic d'une maladie.

12. Utilisation suivant la revendication 11, dans laquelle la maladie est une maladie tumorale, une maladie infectieuse, une maladie de la peau et/ou une maladie du système immunitaire.
